(19) Europäisches Patentamt
European Patent Office
Office européen des brevets

(11) **EP 3 936 529 A1**

(12) **EUROPEAN PATENT APPLICATION**
published in accordance with Art. 153(4) EPC

(43) Date of publication:
**12.01.2022 Bulletin 2022/02**

(51) Int Cl.:
**C08F 8/00** (2006.01)       **C08F 20/06** (2006.01)
**A61F 13/49** (2006.01)       **A61F 13/53** (2006.01)
**B01J 20/26** (2006.01)

(21) Application number: **20769428.2**

(22) Date of filing: **05.03.2020**

(86) International application number:
**PCT/JP2020/009505**

(87) International publication number:
**WO 2020/184393 (17.09.2020 Gazette 2020/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.03.2019 JP 2019042957**

(71) Applicant: **Sumitomo Seika Chemicals Co., Ltd.**
**Kako-gun, Hyogo 675-0145 (JP)**

(72) Inventor: **NISHIDA Moe**
**Himeji-shi, Hyogo 672-8076 (JP)**

(74) Representative: **Nederlandsch Octrooibureau**
**P.O. Box 29720**
**2502 LS The Hague (NL)**

(54) **WATER ABSORBENT RESIN PARTICLES, ABSORBER AND ABSORBENT ARTICLE**

(57)     Water-absorbent resin particles having a degree of gel association of 15 or more, the degree of gel association being measured by the following procedures (1) to (4) are disclosed. (1) 1.0 g of water-absorbent resin particles is disposed in a graduated cylinder having a cylindrical part and a pedestal part, and then 50 ml of pure water is injected to form a swollen gel, (2) a tubular body having a circular opening is prepared, where the tubular body is fixed in a state where both end portions are open in a vertical direction, and a flat portion is provided at an upper end portion, and ten minutes after completion of injection of the pure water, the cylindrical part of the graduated cylinder is inserted into the tubular body so that an opening part of the graduated cylinder faces vertically downward, and thereby the pedestal part of the graduated cylinder is supported by the flat portion of the tubular body, (3) in a state where the tubular body is fixed, the graduated cylinder is freely dropped to cause the pedestal part of the graduated cylinder to collide with the flat portion of the tubular body, and (4) the operation of (3) is performed at intervals of 10 seconds, and the number of times of causing the graduated cylinder to collide with the tubular body until 1 g or more of the swollen gel is dropped is measured as the degree of gel association.

EP 3 936 529 A1

**Description**

**Technical Field**

The present invention relates to water-absorbent resin particles, an absorber, and an absorbent article.

**Background Art**

In the related art, an absorber containing water-absorbent resin particles has been used in an absorbent article for absorbing a liquid having water as a main component (for example, urine). For example,

Patent Literature 1 below discloses water-absorbent resin particles having a particle size that is suitably used for absorbent articles such as diapers. In addition, Patent Literature 2 discloses a method of using a hydrogel-absorbent polymer having specific saline flow inducibility, performance under pressure, and the like as an effective absorbent member for housing a body fluid such as urine.

**Citation List**

**Patent Literature**

[Patent Literature 1] Japanese Unexamined Patent Publication No. H6-345819
[Patent Literature 2] Published Japanese Translation No. H9-510889 of the PCT International Publication

**Summary of Invention**

**Technical Problem**

In an absorbent article, water which has been absorbed by an absorber but is not sufficiently retained (excess water) may be present at an infiltration site of a liquid. In a case where there is a large amount of excess water in the absorber after it absorbs a liquid, problems such as dampness in the absorbent article and liquid adhesion to the skin may occur. Therefore, it is desirable that excess water (excess water content) be reduced in the absorber after it absorbs a liquid.

One aspect of the present invention is to provide water-absorbent resin particles that provide an absorber capable of reducing excess water after it absorbs a liquid. Another aspect of the present invention is to provide an absorber capable of reducing excess water after it absorbs a liquid, and an absorbent article including the absorber.

**Solution to Problem**

One aspect of the present invention provides water-absorbent resin particles having a degree of gel association of 15 or more, the degree of gel association being measured by the following procedures (1) to (4).

(1) 1.0 g of water-absorbent resin particles is disposed in a plastic graduated cylinder, which has a capacity of 100 ml and a mass of 35 g and includes a cylindrical part having an inner diameter of 27 mm and an outer diameter of 31 mm and a pedestal part, and then 50 ml of pure water is injected at a flow rate of 10 ml/sec to form a swollen gel.
(2) An acrylic resin tubular body, which has a circular opening with an inner diameter of 4.5 cm and is fixed in a state where both end portions are open in a vertical direction, is prepared, where a flat portion having a square outer circumference and a height of 1.0 cm is provided at an upper end portion, and the opening is located at a center of the flat portion. Ten minutes after completion of injection of the pure water, the cylindrical part of the graduated cylinder is inserted into the tubular body so that an opening part of the graduated cylinder faces vertically downward, and thereby the pedestal part of the graduated cylinder is supported by the flat portion of the tubular body.
(3) The graduated cylinder is moved vertically upward by 10 cm ± 1 cm in a state where the tubular body is fixed, and thereafter, the graduated cylinder is freely dropped to cause the pedestal part of the graduated cylinder to collide with the flat portion of the tubular body.
(4) The operation of (3) is performed at intervals of 10 seconds, and the number of times of causing the graduated cylinder to collide with the tubular body until 1.0 g or more of the swollen gel is dropped is measured as the degree of gel association.

According to the above-mentioned water-absorbent resin particles, it is possible to obtain an absorber capable of reducing excess water after it absorbs a liquid, and an absorbent article including the absorber.

Another aspect of the present invention provides an absorber containing the above-mentioned water-absorbent resin particles.

Still another aspect of the present invention provides an absorbent article including the above-mentioned absorber.

**Advantageous Effects of Invention**

According to one aspect of the present invention, it is possible to provide water-absorbent resin particles that provide an absorber capable of reducing excess water after it absorbs a liquid. According to another aspect of the present invention, it is possible to provide an absorber capable of reducing excess water after it absorbs a liquid, and an absorbent article including the absorber. According to still another aspect of the present invention, it is possible to provide applications of resin particles, an absorber, and an absorbent article to absorption of a liquid.

**Brief Description of Drawings**

Fig. 1 is a cross-sectional view showing an example of an absorbent article.

Fig. 2 is a plan view showing an outline of a stirring blade (flat plate blade having slits) used in Examples.

Fig. 3 is a schematic view showing a measurement device for a degree of gel association.

Fig. 4 is a schematic view showing a tubular body used for measuring the degree of gel association.

Fig. 5 is a schematic view showing a measurement device for a water absorption amount of water-absorbent resin particles under a load.

**Description of Embodiments**

Hereinafter, embodiments of the present invention will be described in detail. However, the present invention is not limited to the following embodiments, and can be variously modified and implemented within the scope of the gist thereof.

In the present specification, "acrylic" and "methacrylic" are collectively referred to as "(meth)acrylic." "Acrylate" and "methacrylate" are also referred to as "(meth)acrylate." "(Poly)" means both of a case where there is a prefix of "poly" and a case where there is no prefix thereof. Regarding numerical value ranges described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in examples. The term "water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means a total amount of the plurality of substances present in the composition unless otherwise specified. "Physiological saline" refers to an aqueous solution of 0.9% by mass sodium chloride.

In water-absorbent resin particles according to the present embodiment, a degree of gel association measured by the following procedures (1) to (4) is 15 or more. According to the water-absorbent resin particles according to the present embodiment, it is possible to obtain an absorbent article in which excess water after liquid absorption is reduced. According to the present embodiment, it is possible to obtain an absorbent article in which excess water after liquid absorption is reduced but which still has suitable water-absorbent characteristics (water retention amount, water absorption rate, water absorption amount under a load, and the like). In the absorber and the absorbent article which are formed using the water-absorbent resin particles according to the present embodiment, since excess water after liquid absorption is reduced, dampness and liquid adhesion to the skin are inhibited.

(1) 1.0 g of water-absorbent resin particles is disposed in a plastic graduated cylinder, which has a capacity of 100 ml and a mass of 35 g and includes a cylindrical part having an inner diameter of 27 mm and an outer diameter of 31 mm and a pedestal part. Next, 50 ml of pure water is injected into the graduated cylinder at a flow rate of 10 ml/sec to form a swollen gel. In the step (1), regarding the water-absorbent resin particles, the water-absorbent resin particles can be uniformly disposed on a bottom surface inside the graduated cylinder. The injection of pure water can be carried out using a burette. The injection of pure water by the burette is carried out, for example, under the condition in which a distance from the bottom surface inside the graduated cylinder to a distal end of the burette (a liquid dropping port) is 18 cm.

(2) An acrylic resin tubular body, which has a circular opening with an inner diameter of 4.5 cm and is fixed in a state where both end portions are open in a vertical direction, is prepared, where a flat portion having a square outer circumference and a height of 1.0 cm is provided at an upper end portion, and the opening is located at a center of the flat portion. Ten minutes after completion of injection of the pure water in (1), the cylindrical part of the graduated cylinder is inserted into the tubular body so that an opening part of the graduated cylinder faces vertically downward, and thereby the pedestal part of the graduated cylinder is supported by the flat portion of the tubular body.

(3) In a state where the tubular body is fixed, the graduated cylinder is moved vertically upward by 10 cm ± 1 cm with respect to a flat surface (a contact surface of the flat portion of the tubular body with the pedestal part of the graduated cylinder), and thereafter, the graduated cylinder is freely dropped to cause the pedestal part of the graduated cylinder to collide with the flat portion of the tubular body. The step (3) can be carried out by lifting the graduated cylinder vertically upward in a state where the tubular body is fixed using a stand and a clamp, and at a distance of 10 cm ± 1 cm from the flat surface of the tubular body, quickly releasing the graduated cylinder from the hand so that it is freely dropped.

(4) The operation of (3) is performed at intervals of 10 seconds, and the number of times of causing the pedestal part of the graduated cylinder to collide with the tubular body until 1.0 g or more of the swollen gel is dropped is measured as the degree of gel association.

Measurement of a degree of gel association by the above-described procedures (1) to (4) can be specifically performed by a method described in Examples to be described later.

A degree of gel association may be 20 or more, 30 or more, 40 or more, 45 or more, 50 or more, 60 or more, 70 or more, 80 or more, 90 or more, or 100 or more, from the viewpoint of making it easier to obtain an absorber in which excess water is further reduced after liquid absorption. An upper limit of the degree of gel association is not particularly limited, but it may be, for example, 300 or less or 200 or less.

It is sufficient for the water-absorbent resin particles of the present embodiment to be able to retain water, and a liquid that is an absorption target liquid can include water. The water-absorbent resin particles according to the present embodiment are better in absorbability of body fluids such as urine, sweat, and blood (for example, menstrual blood). The water-absorbent resin particles according to the present embodiment can be used as a constituent component of an absorber according to the present embodiment.

A water retention amount of the water-absorbent resin particles according to the present embodiment for physiological saline is preferably within the following range. A water retention amount is preferably 20 g/g or more, 30 g/g or more, 34 g/g or more, 35 g/g or more, 40 g/g or more, 45 g/g or more, 47 g/g or more, or 50 g/g or more, from a viewpoint of easily increasing an absorption capacity of an absorbent article. A water retention amount is preferably 80 g/g or less, 75 g/g or less, 70 g/g or less, 65 g/g or less, 60 g/g or less, or 55 g/g or less, from a viewpoint of easily inhibiting excessive swelling in an absorbent article. From these viewpoints, a water retention amount is preferably 20 to 80 g/g, 30 to 60 g/g, or 47 to 60 g/g. As the water retention amount, a water retention amount at 25°C can be used. The water retention amount can be measured by a method described in Examples to be described later.

A water absorption amount of the water-absorbent resin particles according to the present embodiment for physiological saline under a load is preferably within the following range. A water absorption amount is preferably 8 mL/g or more, 10 mL/g or more, 12 mL/g or more, 15 mL/g or more, 18 mL/g or more, 20 mL/g or more, or 24 mL/g or more, from a viewpoint that a degree of gel association is then likely to become large. A water absorption amount is preferably 40 mL/g or less, 35 mL/g or less, or 30 mL/g or less, from a viewpoint of easily inhibiting excessive swelling in an absorbent article. From these viewpoints, a water absorption amount is preferably 8 to 40 mL/g, 12 to 35 mL/g, or 15 to 30 mL/g. As the water absorption amount for physiological saline under a load, a water absorption amount (25°C) at a load of 4.14 kPa can be used. The water absorption amount can be measured by a method described in Examples to be described later.

A water absorption rate of the water-absorbent resin particles according to the present embodiment for physiological saline is preferably within the following range. A water absorption rate is preferably less than 70 seconds, 65 seconds or less, 60 seconds or less, 57 seconds or less, 55 seconds or less, 50 seconds or less, or 47 seconds or less, from a viewpoint that a liquid is then likely to be suitably absorbed in an absorbent article. A water absorption rate is preferably 20 seconds or more, 25 seconds or more, 30 seconds or more, 35 seconds or more, 38 seconds or more, 40 seconds or more, or 45 seconds or more, from a viewpoint of easily preventing gel blocking caused by a liquid staying in a narrow portion. From these viewpoints, a water absorption rate is preferably 20 to 60 seconds. As the water absorption rate, a water absorption rate at 25°C can be used. The water absorption rate can be measured according to a Vortex method (Japanese Industrial Standard JIS K 7224 (1996)). Specifically, it is possible to obtain the water absorption rate as the time [second] from after the addition of the water-absorbent resin particles until the vortex disappears and the liquid surface becomes flat, when 2.0 ± 0.002 g of the water-absorbent resin particles are added to 50 ± 0.1 g of physiological saline stirred at 600 rpm (rpm = min$^{-1}$) in a 100 mL beaker with a flat bottom surface.

Examples of shapes of the water-absorbent resin particles according to the present embodiment include a substantially spherical shape, a crushed shape, a granular shape, and the like. A median particle size of the water-absorbent resin particles according to the present embodiment may be 250 to 850 $\mu$m, 300 to 700 $\mu$m, or 300 to 600 $\mu$m. The water-absorbent resin particles according to the present embodiment may have a desired particle size distribution at a timing obtained in a production method to be described later, but their particle size distribution may be adjusted by performing operations such as adjustment of a particle size through classification with a sieve.

The water-absorbent resin particles according to the present embodiment can contain, for example, a crosslinking polymer, which are obtained by polymerization of monomers including ethylenically unsaturated monomers, as polymer particles. That is, the water-absorbent resin particles according to the present embodiment can have a structural unit derived from ethylenically unsaturated monomers. As the ethylenically unsaturated monomer, a water-soluble ethylenically unsaturated monomer can be used. Examples of methods of polymerization include a reverse phase suspension polymerization method, an aqueous solution polymerization method, a bulk polymerization method, and a precipitation polymerization method. Among them, the reverse phase suspension polymerization method or the aqueous solution polymerization method is preferable from the viewpoints of facilitating securement of favorable water-absorbent characteristics of the obtained water-absorbent resin particles and control of a polymerization reaction. Hereinbelow, a method for polymerizing ethylenically unsaturated monomers will be described with the reverse phase suspension polymerization method as an example.

An ethylenically unsaturated monomer is preferably water-soluble. Examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quaternarized. The ethylenically unsaturated monomer may be used alone or in a combination of two or more kinds thereof. A functional group, such as a carboxyl group and an amino group, of the monomer, may function as a crosslinkable functional group in a surface crosslinking process to be described later.

Among them, from the viewpoint of high industrial availability, the ethylenically unsaturated monomer preferably includes at least one compound selected from the group consisting of (meth)acrylic acid and a salt thereof, acrylamide, methacrylamide, and N,N-dimethyl acrylamide, and more preferably includes at least one compound selected from the group consisting of (meth)acrylic acid and a salt thereof, and acrylamide. The ethylenically unsaturated monomer more preferably includes at least one compound selected from the group consisting of (meth)acrylic acid and a salt thereof from the viewpoint of further enhancing water-absorbent characteristics (such as a water retention amount). That is, the water-absorbent resin particles preferably have a structural unit derived from at least one selected from the group consisting of (meth)acrylic acid and a salt thereof.

For the monomer for obtaining the water-absorbent resin particles, a monomer other than the above-described ethylenically unsaturated monomers may be used. Such a monomer can be used by, for example, being mixed with an aqueous solution containing the above-described ethylenically unsaturated monomers. A use amount of the ethylenically unsaturated monomer may be 70 to 100 mol%, 80 to 100 mol%, 90 to 100 mol%, 95 to 100 mol%, or 100 mol%, with respect to a total amount of the monomers (the total amount of the monomers for obtaining the water-absorbent resin particles, for example, a total amount of monomers that gives a structural unit of a crosslinking polymer, the same shall apply hereinafter). Among them, a proportion of (meth)acrylic acid and a salt thereof may be 70 to 100 mol%, 80 to 100 mol%, 90 to 100 mol%, 95 to 100 mol%, or 100 mol%, with respect to a total amount of monomers. The "proportion of (meth)acrylic acid and a salt thereof" means a proportion of a total amount of (meth)acrylic acid and a salt thereof.

According to the present embodiment, as an example of the water-absorbent resin particles, it is possible to provide water-absorbent resin particles which contain a crosslinking polymer having a structural unit derived from an ethylenically unsaturated monomer, in which the ethylenically unsaturated monomer includes at least one compound selected from the group consisting of (meth)acrylic acid and a salt thereof, and a proportion of (meth)acrylic acid and a salt thereof is 70 to 100 mol% with respect to a total amount of monomers for obtaining the water-absorbent resin particles.

Usually, the ethylenically unsaturated monomers are suitably used in a form of an aqueous solution. A concentration of the ethylenically unsaturated monomer in an aqueous solution containing the ethylenically unsaturated monomer (hereinafter, simply referred to as a "monomer aqueous solution") is preferably 20% by mass or more and a saturated concentration or less, is more preferably 25% to 70% by mass, and is even more preferably 30% to 55% by mass. Examples of water to be used in an aqueous solution include tap water, distilled water, and ion-exchanged water.

In a case where ethylenically unsaturated monomers have an acid group, a monomer aqueous solution may be used after neutralizing this acid group with an alkaline neutralizing agent. A degree of neutralization of the ethylenically unsaturated monomer by the alkaline neutralizing agent is preferably 10 to 100 mol%, more preferably 50 to 90 mol%, and even more preferably 60 to 80 mol% of the acid group in the ethylenically unsaturated monomer, from a viewpoint of increasing an osmotic pressure of the obtained water-absorbent resin particles, and further enhancing water-absorbent characteristics (water retention amount and the like). Examples of alkaline neutralizing agents include alkali metal salts such as sodium hydroxide, sodium carbonate, sodium hydrogen carbonate, potassium hydroxide, and potassium carbonate; ammonia; and the like. The alkaline neutralizing agent may be used alone or in combination of two or more kinds thereof. The alkaline neutralizing agent may be used in a form of an aqueous solution to simplify a neutralizing operation. Neutralization of the acid groups in the ethylenically unsaturated monomers can be performed by, for example, adding an aqueous solution of sodium hydroxide, potassium hydroxide, or the like dropwise to the above-described monomer aqueous solution and mixing them.

In the reverse phase suspension polymerization method, a monomer aqueous solution can be dispersed in a hydrocarbon dispersion medium in the presence of a surfactant, and polymerization of ethylenically unsaturated monomers can be performed using a radical polymerization initiator or the like. As the radical polymerization initiator, it is possible to use a water-soluble radical polymerization initiator.

Examples of surfactants include nonionic surfactants and anionic surfactants. Examples of nonionic surfactants include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone or in combination of two or more kinds thereof.

The surfactant preferably includes at least one compound selected from the group consisting of sorbitan fatty acid esters, polyglycerin fatty acid esters, and sucrose fatty acid esters, from the viewpoints that then, a state of a W/O type reverse phase suspension becomes favorable, water-absorbent resin particles having a suitable particle size are easily obtained, and industrial availability becomes high. The surfactant preferably includes sucrose fatty acid ester, and more preferably includes sucrose stearic acid ester, from a viewpoint of easily obtaining an appropriate particle size distribution of the water-absorbent resin particles, and from a viewpoint of easily improving water-absorbent characteristics of the water-absorbent resin particles and performances of an absorbent article formed using the same.

A use amount of the surfactant is preferably 0.05 to 10 parts by mass, is more preferably 0.08 to 5 parts by mass, and is even more preferably 0.1 to 3 parts by mass, with respect to 100 parts by mass of the monomer aqueous solution, from the viewpoint that a sufficient effect is obtained within these use amounts, and these amounts are economic.

In the reverse phase suspension polymerization, a polymeric dispersant may be used in combination with the above-mentioned surfactant. Examples of polymeric dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. The polymeric dispersant may be used alone or in combination of two or more kinds thereof. From the viewpoint of better dispersion stability of monomers, the polymeric dispersant is preferably at least one selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer.

A use amount of the polymeric dispersant is preferably 0.05 to 10 parts by mass, is more preferably 0.08 to 5 parts by mass, and is even more preferably 0.1 to 3 parts by mass, with respect to 100 parts by mass of the monomer aqueous solution, from the viewpoint that a sufficient effect is obtained within these use amounts, and these amounts are economic.

The hydrocarbon dispersion medium may include at least one compound selected from the group consisting of a chained aliphatic hydrocarbon having 6 to 8 carbon atoms and an alicyclic hydrocarbon having 6 to 8 carbon atoms. Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone or in combination of two or more kinds thereof.

The hydrocarbon dispersion medium may include at least one selected from the group consisting of n-heptane and cyclohexane from the viewpoints of high industrial availability and stable qualities. Furthermore, from the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A use amount of the hydrocarbon dispersion medium is preferably 30 to 1,000 parts by mass, is more preferably 40 to 500 parts by mass, and is even more preferably 50 to 400 parts by mass, with respect to 100 parts by mass of the monomer aqueous solution, from the viewpoint that polymerization heat is then appropriately removed, and thereby a polymerization temperature is easily controlled. In a case where a use amount of the hydrocarbon dispersion medium is 30 parts by mass or more, a polymerization temperature tends to be easily controlled. In a case where a use amount of the hydrocarbon dispersion medium is 1,000 parts by mass or less, there is a tendency that productivity of polymerization is improved, which is economic.

A radical polymerization initiator is preferably water-soluble. Examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propi onamide}, 2,2'-azobis[2-methyl-N-(2-hydroxyethyl)-propionamide], and 4,4'-azobis(4-cyanovaleric acid). The radical polymerization initiator may be used alone or in combination of two or more kinds thereof. The radical polymerization initiator is preferably at least one selected from the group consisting of potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride.

A use amount of the radical polymerization initiator may be 0.05 to 10 mmol with respect to 1 mole of the ethylenically unsaturated monomer. A case in which a use amount of the radical polymerization initiator is 0.05 mmol or more is efficient, because then a polymerization reaction is not required to be performed for a long period of time. In a case where a use amount of the radical polymerization initiator is 10 mmol or less, it is easy to inhibit occurrence of a rapid polymerization reaction.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, a monomer aqueous solution used for the polymerization may contain a chain transfer agent. Examples of chain transfer agents include hypophosphites, thiols, thiolic acids, secondary alcohols, and amines.

The monomer aqueous solution used for the polymerization may contain a thickener to control a particle size of the water-absorbent resin particles. Examples of thickeners include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, polyethylene oxide, and the like. In a case where stirring speeds in the polymerization are the same, a median particle size of particles to be obtained is likely to become large as a viscosity of the monomer aqueous solution becomes high.

Crosslinking by self-crosslinking may occur during polymerization, and crosslinking may be performed by using an internal crosslinking agent. By using the internal crosslinking agent, it is easy to control water-absorbent characteristics (such as a water retention amount) of the water-absorbent resin particles. The internal crosslinking agent is generally added to a reaction solution in the polymerization reaction. Examples of internal crosslinking agents include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the polyols with unsaturated acids (such as maleic acid and fumaric acid); bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; compounds having two or more reactive functional groups, such as isocyanate compounds (such as 2,4-tolylene diisocyanate and hexamethylene diisocyanate); and the like. The internal crosslinking agent may be used alone or in combination of two or more kinds thereof. The internal crosslinking agent is preferably a polyglycidyl compound, is more preferably a diglycidyl ether compound, and is even more preferably at least one selected from the group consisting of (poly)ethylene glycol diglycidyl ether, (poly)propylene glycol diglycidyl ether, and (poly)glycerin diglycidyl ether.

A use amount of the internal crosslinking agent is preferably 30 mmol or less, more preferably 0.01 to 10 mmol, even more preferably 0.012 to 5 mmol, particularly preferably 0.015 to 1 mmol, extremely preferably 0.02 to 0.1 mmol, and extraordinarily preferably 0.02 to 0.05 mmol, per 1 mole of the ethylenically unsaturated monomer, from the viewpoints that then, a degree of gel association is likely to become large, and water-soluble properties are inhibited by appropriately crosslinking the obtained polymer and thereby a sufficient water absorption amount is easily obtained.

The reverse phase suspension polymerization can be performed in a water-in-oil system by heating, under stirring, ethylenically unsaturated monomers, a radical polymerization initiator, a surfactant, a polymeric dispersant, a hydrocarbon dispersion medium, and the like (and furthermore, an internal crosslinking agent as necessary) in a state where they are mixed.

When performing the reverse phase suspension polymerization, a monomer aqueous solution which contains ethylenically unsaturated monomers is dispersed in a hydrocarbon dispersion medium in the presence of a surfactant (and if necessary, a polymeric dispersant). In this case, a timing of adding the surfactant, the polymeric dispersant, or the like before the start of the polymerization reaction may be either before or after the addition of the monomer aqueous solution.

Among them, it is preferable to carry out the polymerization after dispersing the monomer aqueous solution in the hydrocarbon dispersion medium in which the polymeric dispersant has been dispersed, and then further dispersing the surfactant in the hydrocarbon dispersion medium, from the viewpoint that an amount of the hydrocarbon dispersion medium remaining in the obtained water-absorbent resin can then be easily reduced.

The reverse phase suspension polymerization can be carried out in one stage or in multiple stages of two or more stages. The reverse phase suspension polymerization is preferably carried out in two or three stages from the viewpoint of increasing productivity.

In a case where reverse phase suspension polymerization is carried out in multiple stages of two or more stages, it is sufficient for stages after a second stage of reverse phase suspension polymerization to be carried out in the same manner as in a first stage of reverse phase suspension polymerization by adding ethylenically unsaturated monomers to a reaction mixture obtained in the first stage of polymerization reaction and mixing them, after performing the first stage of reverse phase suspension polymerization. In reverse phase suspension polymerization in each stage after the second stage, reverse phase suspension polymerization is preferably carried out by adding, in addition to ethylenically unsaturated monomers, the above-mentioned radical polymerization initiator and/or internal crosslinking agent within a range of molar ratios of the respective components to the ethylenically unsaturated monomers, based on an amount of ethylenically unsaturated monomers added during reverse phase suspension polymerization in each stage after the second stage. If necessary, the internal crosslinking agent may be used in reverse phase suspension polymerization in each stage after the second stage. In a case where the internal crosslinking agent is used, it is preferable to carry out reverse phase suspension polymerization by adding the internal crosslinking agent within a range of molar ratios of the respective components to the ethylenically unsaturated monomers based on an amount of ethylenically unsaturated monomers provided in each stage.

A temperature for the polymerization reaction varies depending on radical polymerization initiators used, and it is preferably 20°C to 150°C, and is more preferably 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrogel.

After the polymerization, crosslinking may be carried out by adding a post-polymerization crosslinking agent to the obtained hydrous gel polymer and heating them. By performing post-polymerization crosslinking, a degree of crosslinking of the hydrous gel polymer can be increased, and water-absorbent characteristics (such as a water retention amount) can be further improved.

Examples of post-polymerization crosslinking agents include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone or in combination of two or more kinds thereof.

An amount of the post-polymerization crosslinking agent is preferably 30 mmol or less, more preferably 10 mmol or less, even more preferably 0.01 to 5 mmol, particularly preferably 0.012 to 1 mmol, extremely preferably 0.015 to 0.1 mmol, and extraordinarily preferably 0.02 to 0.05 mmol, per 1 mole of the ethylenically unsaturated monomer, from a viewpoint of easily obtaining a suitable degree of gel association and suitable water-absorbent characteristics (such as a water retention amount).

It is sufficient for a timing for adding the post-polymerization crosslinking agent to be after polymerization of ethylenically unsaturated monomers used for the polymerization. In a case of multi-stage polymerization, the crosslinking agent is preferably added after the multi-stage polymerization. From the viewpoint of a water content (to be described later), it is preferable to add the post-polymerization crosslinking agent within a region of [water content immediately after polymerization ± 3% by mass], in consideration of heat generation during and after polymerization, retention due to process delay, system opening when a crosslinking agent is added, and fluctuation in water content due to addition of water associated with addition of a crosslinking agent.

Subsequently, the polymer particles (for example, polymer particles having a structural unit derived from an ethylenically unsaturated monomer) are obtained by drying in order to remove water from the obtained hydrous gel polymer. Examples of drying methods include a method (a) in which a hydrous gel polymer in a state of being dispersed in a hydrocarbon dispersion medium is subjected to azeotropic distillation by heating from the outside, and the hydrocarbon dispersion medium is refluxed to remove water; a method (b) in which a hydrous gel polymer is taken out by decantation and dried under reduced pressure; and a method (c) in which a hydrous gel polymer is separated by filtration with a filter and dried under reduced pressure. Among them, the method (a) is preferably used for its simplicity in production steps.

It is possible to adjust a particle size of the water-absorbent resin particles by adjusting a rotation speed of a stirrer during the polymerization reaction or by adding a flocculating agent to the system after the polymerization reaction or at an initial time of drying. A particle size of the obtained water-absorbent resin particle can be increased by adding the flocculating agent. As the flocculating agent, an inorganic flocculating agent can be used. Examples of inorganic flocculating agents (for example, powdery inorganic flocculating agents) include silica, zeolite, bentonite, aluminum oxide, talc, titanium dioxide, kaolin, clay, and hydrotalcite. The flocculating agent is preferably at least one selected from the group consisting of silica, aluminum oxide, talc, and kaolin from the viewpoint of an better flocculation effect.

In the reverse phase suspension polymerization, a method of adding the flocculating agent is preferably a method in which a flocculating agent is dispersed in a hydrocarbon dispersion medium of the same kind as that used in the polymerization, or water in advance, and then the mixture is mixed into a hydrocarbon dispersion medium containing a hydrous gel polymer under stirring.

An amount of the flocculating agent added is preferably 0.001 to 1 part by mass, is more preferably 0.005 to 0.5 parts by mass, and is even more preferably 0.01 to 0.2 parts by mass, with respect to 100 parts by mass of ethylenically unsaturated monomers used in the polymerization. By setting an amount of the flocculating agent added to be within the above range, it is easy to obtain water-absorbent resin particles having a desired particle size distribution.

In the production of the water-absorbent resin particles, it is preferable to perform surface crosslinking of a surface portion (surface and in the vicinity of surface) of a hydrous gel polymer using a surface crosslinking agent in a drying step (water removing step) or any subsequent steps. By performing surface crosslinking, the water-absorbent characteristics (such as water retention amount) of the water-absorbent resin particles is easily controlled. The surface crosslinking is preferably performed at a timing when the hydrous gel polymer has a specific water content. A timing of the surface crosslinking is preferably a time point at which a water content of the hydrous gel polymer is 5% to 50% by mass, is more preferably a time point at which a water content thereof is 10% to 40% by mass, and is even more preferably a time point at which a water content thereof is 15% to 35% by mass. A water content (% by mass) of the hydrous gel polymer is calculated by the following formula.

$$\text{Water content} = [\text{Ww}/(\text{Ww} + \text{Ws})] \times 100$$

Ww: An amount of water of a hydrous gel polymer obtained by adding an amount of water used, as desired, upon mixing a flocculating agent, a surface crosslinking agent, and the like to an amount obtained by subtracting an amount of water extracted to the outside of the system by the drying process from an amount of water contained in a monomer aqueous solution before polymerization in the all polymerization processes.

Ws: A solid fraction calculated from an amount of materials introduced, such as ethylenically unsaturated monomers, a crosslinking agent, and an initiator, each of which constitutes the hydrous gel polymer.

Examples of surface crosslinking agents include compounds having two or more reactive functional groups. Examples of surface crosslinking agents include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; hydroxyalkylamide compounds such as bis[N,N-di($\beta$-hydroxyethyl)]adipamide; and the like. The surface crosslinking agent may be used alone or in combination of two or more kinds thereof. The surface crosslinking agent is preferably a polyglycidyl compound, and is more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

A use amount of the surface crosslinking agent is preferably 0.01 to 20 mmol, more preferably 0.05 to 10 mmol, even more preferably 0.1 to 5 mmol, particularly preferably 0.15 to 1 mmol, and extremely preferably 0.2 to 0.5 mmol, per 1 mole of the ethylenically unsaturated monomer used for polymerization, from a viewpoint of easily obtaining a suitable degree of gel association and suitable water-absorbent characteristics (such as a water retention amount).

It is possible to obtain polymer particles, which are a surface-crosslinked dried product, by distilling off water and the hydrocarbon dispersion medium by a known method after the surface crosslinking.

As described above, the polymer particles contained in the water-absorbent resin particles can be obtained by using an internal crosslinking agent used at the time of polymerizing the monomer, and can be obtained by using an internal crosslinking agent, and an external crosslinking agent (a post-polymerization crosslinking agent used after the polymerization of the monomer, and a surface c crosslinking agent used in the drying step after polymerization of a monomer or subsequent steps) used after polymerization of the monomer. The ratio of the use amount of the external crosslinking agent with respect to the internal crosslinking agent (external crosslinking agent/internal crosslinking agent) is preferably 5 to 100, more preferably 10 to 80, further more preferably 15 to 50, and particularly preferably 20 to 30, from a viewpoint of easily obtaining a suitable degree of gel association and suitable water-absorbent characteristics (such as water retention amount). The water-absorbent resin particles may contain polymer particles which are reaction products using an internal crosslinking agent, and may contain polymer particles which are reaction products using an internal crosslinking agent and an external crosslinking agent. A ratio of the use amount of the external crosslinking agent to the internal crosslinking agent in the polymer particles is preferably in the above range.

The polymerization reaction can be carried out using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, and the like. A flat plate blade has a shaft (stirring shaft) and a flat plate portion (stirring portion) disposed around the shaft. The flat plate portion may have a slit or the like. In a case where the flat plate blade is used as the stirring blade, it is easy to uniformly carry out the crosslinking reaction in polymer particles, and it is easy to increase a degree of gel association to the extent suitable for the invention of the present application while maintaining water-absorbent characteristics such as a water retention amount.

In addition to the polymer particles, the water-absorbent resin particles according to the present embodiment can further contain, for example, various additional such as gel stabilizers, metal chelating agents (ethylenediaminetetraacetic acid and its salts, diethylenetriaminepentaacetic acid and its salts, for example, diethylenetriaminepentaacetic acid pentasodium, and the like), and flowability improvers (lubricants). The additional components may be disposed inside the polymer particles, on a surface of the polymer particles, or both of the inside and on the surface thereof.

The water-absorbent resin particles may contain a plurality of inorganic particles disposed on the surface of the polymer particles. The inorganic particles can be disposed on the surface of the polymer particles by, for example, mixing the polymer particles and the inorganic particles. These inorganic particles may be silica particles such as amorphous silica.

In a case where the water-absorbent resin particles include inorganic particles disposed on the surface of the polymer particles, a content of the inorganic particles may be within the following range based on a total mass of the polymer particles. A content of the inorganic particles may be 0.05% by mass or more, 0.1% by mass or more, 0.15% by mass or more, or 0.2% by mass or more. A content of the inorganic particles may be 5.0% by mass or less, 3.0% by mass or less, 1.0% by mass or less, 0.5% by mass or less, or 0.3% by mass or less.

The inorganic particles referred to herein generally have a minute size as compared with a size of the polymer particles. For example, an average particle size of the inorganic particles may be 0.1 to 50 $\mu$m, 0.5 to 30 $\mu$m, or 1 to 20 $\mu$m. The average particle size can be measured by a pore electric resistance method or a laser diffraction/scattering method depending on the characteristics of the particles.

An absorber according to the present embodiment contains the water-absorbent resin particles according to the present embodiment. The absorber according to the present embodiment may contain a fibrous substance, and it is, for example, a mixture containing the water-absorbent resin particles and the fibrous substance. The configuration of the absorber may be, for example, a configuration in which water-absorbent resin particles and the fibrous substances are uniformly mixed, a configuration in which water-absorbent resin particles are held between fibrous substances formed in a sheet shape or a layer shape, or another configuration.

Examples of fibrous substances include finely pulverized wood pulp; cotton; cotton linter; rayon; cellulose-based fibers such as cellulose acetate; synthetic fibers such as polyamides, polyesters, and polyolefins; a mixture of these fibers; and the like. The fibrous substance may be used alone or in combination of two or more kinds thereof. As the fibrous substance, hydrophilic fibers can be used.

Fibers may be adhered to each other by adding an adhesive binder to the fibrous substance in order to enhance shape retention properties before or during use of the absorber. Examples of adhesive binders include heat-sealing synthetic fibers, hot melt adhesives, and adhesive emulsions. The adhesive binder may be used alone or in combination of two or more kinds thereof.

Examples of heat-sealing synthetic fibers include full-melt binders such as polyethylene, polypropylene, and an ethylene-propylene copolymer; and partial-melt binders formed of polypropylene and polyethylene in a side-by-side or core-and-sheath configuration. In the above-mentioned partial-melt binders, only a polyethylene portion can be thermal-bonded.

Examples of hot melt adhesives include a mixture of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a viscosity imparting agent, a plasticizer, an antioxidant, or the like.

Examples of adhesive emulsions include polymers of at least one monomer selected from the group consisting of methyl methacrylate, styrene, acrylonitrile, 2-ethylhexyl acrylate, butyl acrylate, butadiene, ethylene, and vinyl acetate.

The absorber according to the present embodiment may contain inorganic powders (for example, amorphous silica), deodorants, antibacterial agents, pigments, dyes, fragrances, pressure sensitive adhesives, and the like. In a case where the water-absorbent resin particles contain inorganic particles, the absorber may contain an inorganic powder in addition to the inorganic particles in the water-absorbent resin particles.

A shape of the absorber according to the present embodiment may be, for example, a sheet shape. A thickness of the absorber (for example, a thickness of a sheet-shaped absorber) may be 0.1 to 20 mm or 0.3 to 15 mm.

A content of the water-absorbent resin particles in the absorber may be 2% to 100% by mass, 10% to 80% by mass, or 20% to 60% by mass, with respect to a total of the water-absorbent resin particles and the fibrous substance, from a viewpoint of easily obtaining sufficient water-absorbent characteristics.

A content of the water-absorbent resin particles in the absorber is preferably 100 to 1,000 g, is more preferably 150 to 800 g, and is even more preferably 200 to 700 g, per 1 $m^2$ of the absorber from the viewpoint of easily obtaining sufficient water-absorbent characteristics. A content of the fibrous substance in the absorber is preferably 50 to 800 g, is more preferably 100 to 600 g, and is even more preferably 150 to 500 g, per 1 $m^2$ of the absorber from the viewpoint of easily obtaining sufficient water-absorbent characteristics.

An absorbent article according to the present embodiment includes the absorber according to the present embodiment. Examples of other constituent members of the absorbent article according to the present embodiment include a core wrap that retains the shape of the absorber and also prevents the constituent members of the absorber from falling off or flowing; a liquid permeable sheet disposed on the outermost part on a side from which an absorption target liquid is infiltrated; a liquid impermeable sheet disposed on the outermost part on a side opposite to the side from which the absorption target liquid is infiltrated; and the like. Examples of the absorbent article include diapers (for example, paper diapers), toilet training pants, incontinence pads, hygiene products (sanitary napkins, tampons, and the like), sweat pads, pet sheets, portable toilet members, animal excrement treatment materials, and the like.

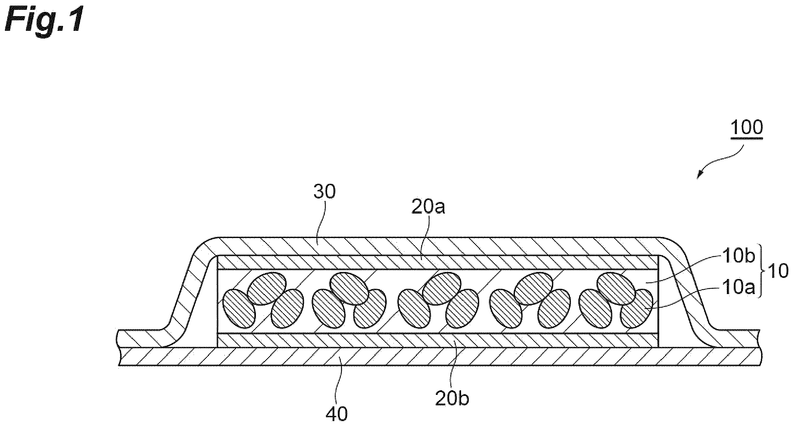

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown to be a gap between the members, but the members may be in close contact with each other without the gap.

The absorber 10 has water-absorbent resin particles 10a according to the present embodiment and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 1) in a state of being in contact with the absorber 10. The core wrap 20b is disposed on the other surface side of the absorber 10 (a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, net-like sheets having a mesh, and the like. The core wrap 20a and the core wrap 20b each have, for example, a main surface having the same size as that of the absorber 10.

The liquid permeable sheet 30 is disposed on the outermost part on a side from which an absorption target liquid is infiltrated. The liquid permeable sheet 30 is disposed on the core wrap 20a in a state of being in contact with the core wrap 20a. Examples of the liquid permeable sheet 30 include non-woven fabrics made of synthetic resin such as polyethylene, polypropylene, polyester, and polyamide, and porous sheets. The liquid impermeable sheet 40 is disposed on the outermost part on a side opposite to the liquid permeable sheet 30, in the absorbent article 100. The liquid impermeable sheet 40 is disposed below the core wrap 20b in a state of being in contact with the core wrap 20b. Examples of the liquid impermeable sheet 40 include sheets made of synthetic resins such as polyethylene, polypropylene, and polyvinyl chloride, and sheets made of a composite material of these synthetic resins and a non-woven fabric. The liquid permeable sheet 30 and the liquid impermeable sheet 40 each have, for example, a main surface wider than the main surface of the absorber 10, and outer edges of the liquid permeable sheet 30 and the liquid impermeable sheet 40 respectively extend around the absorber 10 and the core wraps 20a and 20b.

A magnitude relationship between the absorber 10, the core wraps 20a and 20b, the liquid permeable sheet 30, and the liquid impermeable sheet 40 is not particularly limited, and it is appropriately adjusted according to usage applications and the like of the absorbent article. Furthermore, a method of retaining the shape of the absorber 10 using the core wraps 20a and 20b is not particularly limited. The absorber may be wrapped with a plurality of the core wraps as shown in Fig. 1, or the absorber may be wrapped with one core wrap.

The absorber may be adhered to the top sheet. In a case where the absorber is sandwiched or covered by the core wrap, it is preferable that at least the core wrap and the top sheet be adhered to each other, and it is more preferable that the core wrap and the absorber be adhered to each other in addition to the adhesion of the core wrap and the top sheet. Examples of methods of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains heat-sealing synthetic fibers, a method of adhering by heat-sealing of the heat-sealing synthetic fibers may be adopted.

According to the present embodiment, it is possible to provide a method of absorbing a liquid using the water-absorbent resin particles, absorber, or absorbent article according to the present embodiment. The method of absorbing a liquid according to the present embodiment includes a step of bringing an absorption target liquid into contact with the water-absorbent resin particles, absorber, or absorbent article according to the present embodiment.

According to the present embodiment, it is possible to provide a method of reducing excess water when the absorber absorbs a liquid, which is a method using the water-absorbent resin particles, absorber, or absorbent article according to the present embodiment. According to the present embodiment, it is possible to provide a method of reducing excess water when the absorber absorbs a liquid by using the above-described water-absorbent resin particles in which a degree of gel association is 15 or more. The method of reducing excess water when the absorber according to the present embodiment absorbs a liquid includes an adjustment step of adjusting a degree of gel association measured by the above-described procedures relating to the water-absorbent resin particles according to the present embodiment. In the adjustment step, a degree of gel association can be adjusted to be within each of the above-described ranges (for example, 15 or more).

According to the present embodiment, it is possible to provide a method for producing water-absorbent resin particles, the method including a selection step of selecting water-absorbent resin particles based on the above-mentioned degree of gel association. In the selection step, for example, water-absorbent resin particles are selected based on whether or not the above-mentioned degree of gel association is 15 or more. That is, according to one embodiment, it is possible to provide a method for producing water-absorbent resin particles, the method including a selection step of selecting water-absorbent resin particles in which a degree of gel association is 15 or more.

According to the present embodiment, it is possible to provide a method of reducing excess water when the absorber absorbs a liquid, the method including increasing the above-mentioned degree of gel association of the water-absorbent resin particles.

According to the present embodiment, it is possible to provide a method for producing an absorber by using the water-absorbent resin particles obtained by the above-mentioned method for producing water-absorbent resin particles. The method for producing an absorber according to the present embodiment includes a particle producing step of obtaining water-absorbent resin particles by the above-described method for producing water-absorbent resin particles. The method for producing an absorber according to the present embodiment may include a step of mixing the water-absorbent resin particles and a fibrous substance after the particle producing step. According to the present embodiment, it is possible to provide a method for producing an absorbent article by using the absorber obtained by the above-described method for producing an absorber. The method for producing an absorbent article according to the present embodiment includes an absorber producing step of obtaining an absorber by the above-described method for producing an absorber. The method for producing an absorbent article according to the present embodiment may include a step of obtaining an absorbent article by using the absorber and other constituent members for an absorbent article after the absorber producing step. In this step, for example, an absorbent article is obtained by laminating the absorber and other constituent members for an absorbent article with each other.

**Examples**

Hereinafter, contents of the present invention will be described in further detail using examples and comparative examples, but the present invention is not limited to the following examples.

<Production of water-absorbent resin particles>

(Example 1)

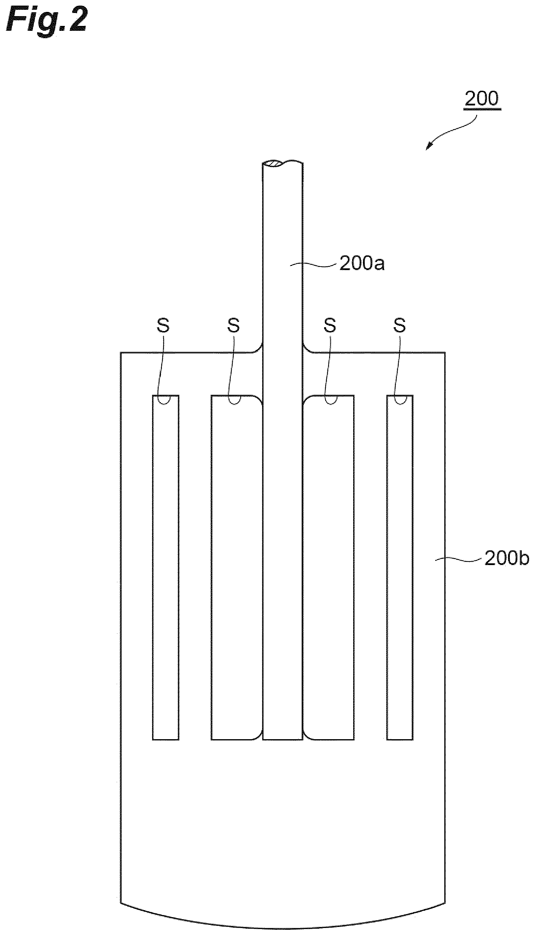

A cylindrical round-bottomed separable flask with the inner diameter of 11 cm and the internal volume of 2 L equipped with a reflux condenser, a dropping funnel, a nitrogen gas introduction tube, and a stirrer was prepared. The stirrer was equipped with a stirring blade (flat plate blade) 200 of which an outline is shown in Fig. 2. The stirring blade 200 includes a shaft 200a and a flat plate portion 200b. The flat plate portion 200b is welded to the shaft 200a and has a curved distal end. The flat plate portion 200b is formed with four slits S extending along an axial direction of the shaft 200a. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. Subsequently, 293 g of n-heptane as a hydrocarbon dispersion medium was added into the above-mentioned separable flask, and 0.736 g of a maleic anhydride-modified ethylene-propylene copolymer (HI-WAX 1105A, manufactured by Mitsui Chemicals, Inc.) as a polymeric dispersant was added thereinto. Thereby, a mixture was obtained. The dispersant was dissolved by raising the temperature to 80°C while stirring the mixture, and then the mixture was cooled to 50°C.

Subsequently, 92.0 g of an aqueous solution of 80.5% by mass acrylic acid (acrylic acid: 1.03 mole) was added into a beaker having the internal volume of 300 mL as a water-soluble ethylenically unsaturated monomer. Subsequently, while cooling the beaker from the outside, 147.7 g of an aqueous solution of 20.9% by mass sodium hydroxide was added dropwise into the beaker to perform 75 mol% of neutralization of acrylic acid. Thereafter, 0.092 g of hydroxy ethyl cellulose (HEC AW-15F, manufactured by Sumitomo Seika Chemicals Co., Ltd.) as a thickener, 0.0736 g (0.272 mmol) of potassium persulfate as a water-soluble radical polymerization initiator, and 0.010 g (0.057 mmol) of ethylene glycol diglycidyl ether as an internal crosslinking agent were added and then dissolved. Thereby, a first stage aqueous liquid was prepared.

Then, the first stage aqueous liquid was added into the above-mentioned separable flask, and then stirring was performed for 10 minutes. Thereafter, 0.736 g of sucrose stearic acid ester (surfactant, Mitsubishi-Chemical Foods Corporation, RYOTO Sugar Ester S-370, HLB value: 3) was dissolved in 6.62 g of n-heptane by heating, and thereby a surfactant solution was obtained. This surfactant solution was added into the separable flask. Then, the inside of the system was sufficiently replaced with nitrogen while stirring at the rotation speed of 425 rpm of the stirrer. Thereafter, the flask was immersed in a water bath at 70°C to raise its temperature, and polymerization was performed for 60 minutes. Thereby, a first stage polymerization slurry solution was obtained.

Subsequently, 128.8 g of an aqueous solution of 80.5% by mass acrylic acid (acrylic acid: 1.44 mole) was added into another beaker having the internal volume of 500 mL as a water-soluble ethylenically unsaturated monomer. Subsequently, while cooling the beaker from the outside, 159.0 g of an aqueous solution of 27% by mass sodium hydroxide was added dropwise into the beaker to perform 75 mol% of neutralization of acrylic acid. Thereafter, 0.090 g (0.333 mmol) of potassium persulfate as a water-soluble radical polymerization initiator, and 0.0116 g (0.067 mmol) of ethylene glycol diglycidyl ether as an internal crosslinking agent were added and then dissolved. Thereby, a second stage aqueous liquid was prepared.

Subsequently, while stirring at the rotation speed of 650 rpm of the stirrer, the inside of the above-mentioned separable flask was cooled to 25°C, and then a total amount of the above-mentioned second stage aqueous liquid was added to the above-mentioned first stage polymerization slurry solution. Subsequently, after replacing the inside of the system with nitrogen for 30 minutes, the flask was immersed in a water bath at 70°C again to raise the temperature, and the polymerization reaction was performed for 60 minutes. Accordingly, a second stage hydrous gel polymer was obtained.

Under stirring, 0.589 g of an aqueous solution of 45% by mass diethylenetriaminepentaacetic acid pentasodium was added to the above-mentioned second stage hydrous gel polymer. Thereafter, the flask was immersed in an oil bath set to 125°C, and 271.4 g of water was extracted out of the system while n-heptane was refluxed by azeotropic distillation with n-heptane and water. Then, 4.42 g of an aqueous solution of 2% by mass ethylene glycol diglycidyl ether (ethylene glycol diglycidyl ether: 0.507 mmol) was added into the flask as a surface crosslinking agent, and then the mixture was maintained at 83°C for 2 hours.

Thereafter, n-heptane was evaporated at 125°C, and the residue was dried to obtain polymer particles (dried product). After passing these polymer particles through a sieve having the opening of 850 μm, 0.2% by mass of amorphous silica (Tokusil NP-S manufactured by Oriental Silicas Corporation) was mixed with the polymer particles based on a total mass of the polymer particles to obtain 232.0 g of water-absorbent resin particles containing amorphous silica. A median particle size of the water-absorbent resin particles was 359 μm.

(Example 2)

231.0 g of water-absorbent resin particles was obtained in the same manner as in Example 1 except that, in preparation of a first stage aqueous liquid, 0.092 g (0.339 mmol) of 2,2'-azobis(2-amidinopropane) dihydrochloride and 0.018 g (0.067 mmol) of potassium persulfate as a water-soluble radical polymerization initiator, and 0.0046 g (0.026 mmol) of ethylene glycol diglycidyl ether as an internal crosslinking agent were used; in preparation of a second stage aqueous liquid, 0.129 g (0.476 mmol) of 2,2'-azobis(2-amidinopropane) dihydrochloride and 0.026 g (0.096 mmol) of potassium persulfate as a water-soluble radical polymerization initiator were used; and for a second stage hydrous gel polymer, 217.8 g of water was extracted out of the system by azeotropic distillation. A median particle size of the water-absorbent resin particles was 339 μm.

(Example 3)

232.1 g of water-absorbent resin particles was obtained in the same manner as in Example 1 except that, in preparation of a first stage aqueous liquid, 0.092 g (0.339 mmol) of 2,2'-azobis(2-amidinopropane) dihydrochloride and 0.018 g (0.067 mmol) of potassium persulfate were used as a water-soluble radical polymerization initiator, and 0.0046 g (0.026 mmol) of ethylene glycol diglycidyl ether was used as an internal crosslinking agent; in preparation of a second stage aqueous liquid, 0.129 g (0.476 mmol) of 2,2'-azobis(2-amidinopropane) dihydrochloride and 0.026 g (0.096 mmol) of potassium persulfate as a water-soluble radical polymerization initiator were used; and for a second stage hydrous gel polymer, 234.6 g of water was extracted out of the system by azeotropic distillation. A median particle size of the water-absorbent resin particles was 355 μm.

(Comparative Example 1)

231.0 g of water-absorbent resin particles was obtained in the same manner as in Example 1 except that, a stirring blade having four inclined paddle blades, each having a blade diameter of 5 cm, in a two-tier manner was used as a stirring blade; in preparation of a first stage polymerization slurry solution, a rotation speed of the stirrer was set to 550 rpm; in production of a second stage hydrous gel polymer, a rotation speed of the stirrer was set to 1,000 rpm; 0.265 g of an aqueous solution of 45% by mass diethylenetriaminepentaacetic acid pentasodium was added to the second stage hydrous gel polymer; for the second stage hydrous gel polymer, 247.9 g of water was extracted out of the system by azeotropic distillation; and 0.5% by mass of amorphous silica with respect to a mass of polymer particles was mixed with the polymer particles. A median particle size of the water-absorbent resin particles was 355 μm.

(Comparative Example 2)

230.8 g of water-absorbent resin particles were obtained in the same manner as that in Comparative Example 1, except that, in the hydrous gel polymer obtained after the second stage polymerization, 256.1 g of water was extracted to the outside of the system by azeotropic distillation; and 0.1% by mass of amorphous silica with respect to a mass of polymer particles was mixed with the polymer particles. A median particle size of the water-absorbent resin particles was 349 μm.

(Comparative Example 3)

224.6 g of water-absorbent resin particles was obtained in the same manner as in Comparative Example 1 except that in preparation of a first stage aqueous liquid, 0.092 g (0.339 mmol) of 2,2'-azobis(2-amidinopropane) dihydrochloride and 0.018 g (0.067 mmol) of potassium persulfate were used as a water-soluble radical polymerization initiator, and 0.0046 g (0.026 mmol) of ethylene glycol diglycidyl ether was used as an internal crosslinking agent; in preparation of a second stage aqueous liquid, 0.129 g (0.476 mmol) of 2,2'-azobis(2-amidinopropane) dihydrochloride and 0.026 g (0.096 mmol) of potassium persulfate were used as a water-soluble radical polymerization initiator, and 0.0116 g (0.067 mmol) of ethylene glycol diglycidyl ether was used as an internal crosslinking agent; 0.589 g of an aqueous solution of 45% by mass diethylenetriaminepentaacetic acid pentasodium was added to a second stage hydrous gel polymer; for the hydrous gel polymer obtained after second stage polymerization, 209.7 g of water was extracted out of the system by azeotropic distillation, and 6.62 g (0.761 mmol) of an aqueous solution of 2% by mass ethylene glycol diglycidyl ether was used as a surface crosslinking agent; and 0.2% by mass of amorphous silica with respect to a mass of polymer particles was mixed with the polymer particles. The median particle size of the water-absorbent resin particles was 356 μm.

(Comparative Example 4)

225.6 g of water-absorbent resin particles was obtained in the same manner as in Comparative Example 3 except that, for a hydrous gel polymer obtained after second stage polymerization, 207.9 g of water was extracted out of the system by azeotropic distillation, and 4.42 g (0.507 mmol) of an aqueous solution of 2% by mass ethylene glycol diglycidyl ether was used as a surface crosslinking agent. A median particle size of the water-absorbent resin particles was 361 μm.

<Measurement of median particle size>

The above-mentioned median particle size of the water-absorbent resin particles was measured by the following procedure. That is, JIS standard sieves were combined in the following order from the top: a sieve having an opening of 600 μm, a sieve having an opening of 500 μm, a sieve having an opening of 425 μm, a sieve having an opening of 300 μm, a sieve having an opening of 250 μm, a sieve having an opening of 180 μm, a sieve having an opening of 150 μm, and a saucer. 50 g of the water-absorbent resin particles was fed to the topmost sieve among the combination of the sieves, and using a Ro-Tap shaker (manufactured by Iida-seisakusho Japan Corporation), classification was performed according to JIS Z 8815 (1994). After the classification, a mass of the particles remaining on each of the sieves was calculated as a mass percentage with respect to a total amount to determine a particle size distribution. By integrating values on the sieves in descending order of the particle sizes with regard to the particle size distribution, a relationship between the opening of the sieve and the integrated value of mass percentages of the particles remaining on the sieve was plotted on a log-probability paper. The plotted points on the probability paper were connected with straight lines, and a particle size corresponding to 50% by mass of the integrated mass percentage was obtained as a median particle size.

<Measurement of degree of gel association>

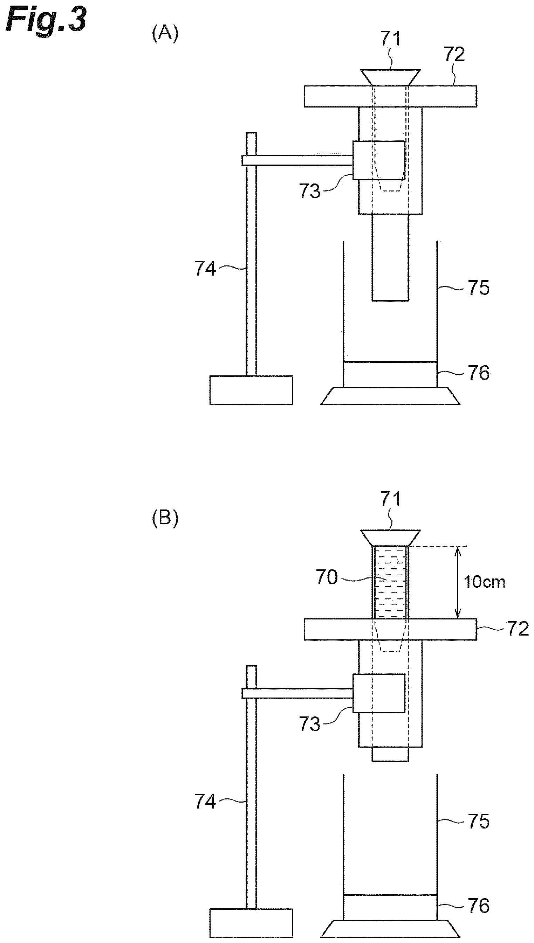

Fig. 3 is a schematic view showing a measurement device for a degree of gel association. The measurement device shown in Fig. 3 has a graduated cylinder 71, a tubular body 72, a clamp 73, a stand 74, a saucer 75, and a balance 76. The saucer 75 is placed on the balance 76. The saucer 75 and the balance 76 are disposed below the graduated cylinder 71 and the tubular body 72.

The graduated cylinder 71 is a plastic graduated cylinder (manufacturer: Kartell) having a capacity of 100 ml and a mass of 35 g and including a cylindrical part a pedestal part. This cylindrical part has an inner diameter of 27 mm and an outer diameter of 31 mm. Furthermore, the pedestal part is a regular pentagon with a side of 38 mm. A specific material of the graduated cylinder is polymethylpentene, and a total height (height of the cylindrical part and the pedestal part) is 249 mm.

A swollen gel 70 is formed in the graduated cylinder 71. The swollen gel 70 is produced by disposing 1.0 ± 0.001 g of water-absorbent resin particles in the graduated cylinder 71, and then injecting 50 ml of pure water using a burette adjusted to a flow rate of 10 ml/sec. In the production of the swollen gel 70, a distance from the bottom of the graduated cylinder 71 to a distal end of the burette (a liquid dropping port) when injecting pure water was set to 18 cm.

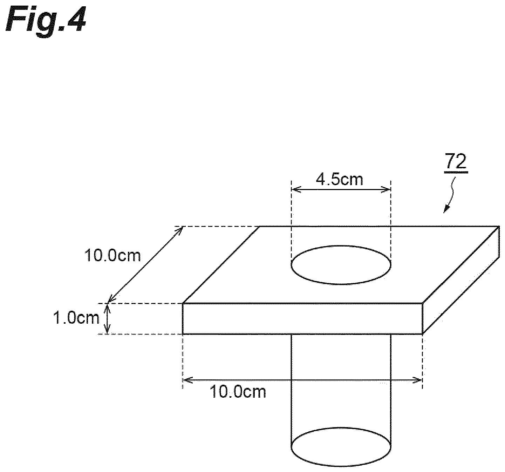

The tubular body 72 is an acrylic resin tubular body shown in Fig. 4. The tubular body 72 has a circular opening with an inner diameter of 4.5 cm. The tubular body 72 has a square shape with an outer circumference of 10 cm on a side, and is provided with a flat surface in which the opening is located at the center of the flat surface. The tubular body 72 is configured of a flat portion having a flat surface and a cylindrical portion. A height (thickness) of the flat portion is 1.0 cm, and a height of the cylindrical portion is 6.0 cm. As shown in Fig. 3, the tubular body 72 is fixed by the stand 74 and the clamp 73 so that the flat surface is located on the upper end side in a state where the tubular body is open in the vertical direction.

Ten minutes after the completion of injection of pure water into the graduated cylinder 71 in which the water-absorbent resin particles were disposed in the production of the swollen gel 70, the cylindrical part of the graduated cylinder 71 was inserted from the flat portion side to the opening of the tubular body 72 so that an opening part of the graduated cylinder 71 faced vertically downward. Accordingly, as shown in Fig. 3(A), the pedestal part of the graduated cylinder 71 was supported by the flat portion of the tubular body 72. Next, as shown in Fig. 3(B), in a state where the tubular body 72 was fixed, the graduated cylinder 71 was lifted vertically upward by 10 cm ± 1 cm with respect to a flat surface of the tubular body 72 (a contact surface of the flat portion of the tubular body 72 with the pedestal part of the graduated cylinder 71). Thereafter, by releasing the graduated cylinder 71 from the hand to cause the graduated cylinder 71 to be freely dropped, the graduated cylinder 71 and the tubular body 72 collided with each other to apply an impact. The operation of applying an impact by lifting the graduated cylinder 71 and releasing it from the hand was repeated at intervals of 10 seconds, and the number of times of applying the impact until 1.0 g or more of the swollen gel 70 was dropped was measured as a degree of gel association. Amass (g) of the swollen gel 70 recovered in the saucer 75 was measured with a balance 76, and the number of times of applying the impact until the amount thereof reached 1.0 g or more was recorded as a degree of gel association (unit: times). In a case where a mass of the swollen gel 70 recovered in the saucer 75 was less than 1.0 g when the impact was applied 100 times, this case was evaluated as a degree of gel association of 100 or more. Measurement of the degree of gel association was performed three times, and an average value of the three times of the measurements was taken as a degree of gel association. The results are shown in Table 1.

<Water retention amount of water-absorbent resin particles>

A water retention amount (room temperature, 25°C ± 2°C) of the water-absorbent resin particles for physiological saline was measured by the following procedure. First, a cotton bag (cotton broadcloth No. 60, 100 mm in width × 200 mm in length) into which 2.0 g of the water-absorbent resin particles had been weighed was placed in a beaker having an internal volume of 500 mL. 500 g of an aqueous solution of 0.9% by mass sodium chloride (physiological saline) was poured into the cotton bag containing the water-absorbent resin particles at once so that a lump could not be produced. Thereafter, the upper part of the cotton bag was bound with a rubber band and left to stand for 30 minutes, and thereby the water-absorbent resin particles were swollen. The cotton bag after an elapse of 30 minutes was dehydrated for 1 minute using a dehydrator (manufactured by KOKUSAN Co., Ltd., product number: H-122) which had been set at a centrifugal force of 167 G. Thereafter, a mass Wa [g] of the dehydrated cotton bag containing the swollen gel was measured. By performing the same operation without addition of the water-absorbent resin particles, a mass Wb [g] of an empty cotton bag upon moisturizing was measured, and a water retention amount of the water-absorbent resin particles for physiological saline was calculated from the following formula. The results are shown in Table 1.

$$\text{Water retention amount } [\text{g/g}] = (Wa - Wb)/2.0$$

<Water absorption amount of water-absorbent resin particles under load>

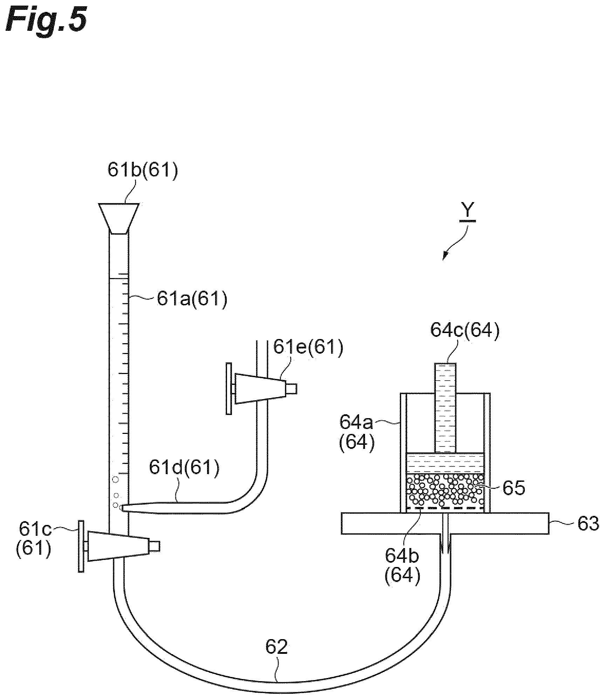

A water absorption amount (room temperature, 25°C ± 2°C) of the water-absorbent resin particles for physiological saline under a load (under pressurization) was measured using a measurement device Y shown in Fig. 5. The measurement device Y is composed of a burette unit 61, a conduit 62, a measurement table 63, and a measurement unit 64 placed on the measurement table 63. The burette unit 61 includes a burette 61a extending in a vertical direction, a rubber stopper 61b disposed at an upper end of the burette 61a, a cock 61c disposed at a lower end of the burette 61a, an air introduction tube 61d having one end extending to the burette 61a in the vicinity of the cock 61c, and a cock 61e disposed at the other end side of the air introduction tube 61d. The conduit 62 is attached between the burette unit 61 and the measurement table 63. An inner diameter of the conduit 62 is 6 mm. A hole with a diameter of 2 mm is present in a central portion of the measurement table 63, and the conduit 62 is connected to the hole. The measurement unit 64 includes a cylinder 64a (made of acrylic resin (plexiglass)), a nylon mesh 64b adhered to a bottom portion of the cylinder 64a, and a weight 64c. An inner diameter of the cylinder 64a is 20 mm. An opening of the nylon mesh 64b is 75 μm (200 mesh). Then, at the time of measurement, water-absorbent resin particles 65 which are measurement targets are uniformly sprinkled on the nylon mesh 64b. A diameter of the weight 64c is 19 mm, and a mass of the weight 64c is 119.6 g. The weight 64c can be placed on the water-absorbent resin particles 65 to apply a load of 4.14 kPa to the water-absorbent resin particles 65.
After 0.100 g of the water-absorbent resin particles 65 were put into the cylinder 64a of the measurement device Y, the weight 64c was placed thereon, and the measurement was started. The same volume of air as the physiological saline absorbed by the water-absorbent resin particles 65 was quickly and smoothly supplied to the inside of the burette 61a through the air introduction tube, and therefore a reduced amount in water level of the physiological saline inside the burette 61a was an amount of the physiological saline absorbed by the water-absorbent resin particles 65. A scale of the burette 61a was engraved from 0 mL in increments of 0.5 mL, from the top to bottom direction. A scale Va of the burette 61a before the start of water absorption and a scale Vb of the burette 61a 60 minutes after the start of water absorption were read as water levels of the physiological saline, and a water absorption amount under a load was calculated from the following formula. The results are shown in Table 1.

$$\text{Water absorption amount under pressure } [\text{mL/g}] = (Vb - Va)/0.1$$

<Water absorption rate of water-absorbent resin particles>

A water absorption rate of the water-absorbent resin particles for physiological saline was measured by the following procedure based on the Vortex method. First, 50 ± 0.1 g of an aqueous solution of 0.9% by mass sodium chloride (physiological saline) adjusted to the temperature of 25°C ± 0.2°C in a thermostatic water bath was weighed into a beaker having the internal volume of 100 mL. Subsequently, a vortex was generated by stirring at the rotation speed of 600 rpm using a magnetic stirrer bar (8 mmφ × 30 mm, without ring). 2.0 ± 0.002 g of the water-absorbent resin particles were added to the aqueous solution of sodium chloride at one time. The time [seconds] from after the addition of the water-absorbent resin particles until the vortex on the liquid surface converged was measured, and this time was obtained as the water absorption rate of the water-absorbent resin particles. The results are shown in Table 1.

<Evaluation of absorber performance>

(Production of article for evaluation)

10 g of the water-absorbent resin particles and 10 g of pulverized pulp were uniformly mixed by air papermaking using an air flow type mixer (Padformer manufactured by OTEC Co., Ltd.), and thereby a sheet-shaped absorber having a size of 40 cm × 12 cm was produced. Subsequently, the absorber was disposed on a core wrap (tissue paper) having the same size as that of the absorber and having the basis weight of 16 g/m$^2$, and then a core wrap (tissue paper) having the same size as that of the absorber and having the basis weight of 16 g/m$^2$ was disposed on an upper surface of the absorber. A laminate was obtained by applying the load of 141 kPa to the absorber sandwiched by the core wraps for 30 seconds. An SMMS non-woven fabric having a size of 32.5 cm × 45.0 cm (basis weight of 13 g/m$^2$) was folded to a size of 45 cm × 16.25 cm, and thereafter, the above-mentioned laminate was wrapped with the non-woven fabric. Three sides of the non-woven fabric that wrapped the laminate were crimped by a heat sealer (Fuji Impulse Sealer, model number: FI-450-5 type, manufactured by FUJI IMPULSE CO., LTD.) to seal the laminate. Accordingly, an article for evaluation was obtained.

(Measurement of water absorption amount of absorber)

A wire mesh (opening size: 20 mm × 20 mm, linear 3 mm) and 20 L of an aqueous solution of 0.9% by mass sodium chloride were put in a vat, and a liquid temperature was adjusted to 25.0°C ± 0.2°C. Next, the article for evaluation was disposed on the wire mesh and immersed in the aqueous solution of 0.9% by mass sodium chloride for 30 minutes. Thereafter, the wire mesh was lifted together with the article for evaluation, drained for 5 minutes, and then a mass of the article for evaluation was measured. A water absorption amount (unit: g) of the absorber is a difference in masses of the article for evaluation before and after the test, and is calculated by the following formula.

$$\text{Water absorption amount of absorber} = \text{mass (g) of article for evaluation after test} - \text{mass (g) of article for evaluation before test}$$

(Measurement of water retention amount of absorber)

The article for evaluation (article for evaluation after the test) for which the water absorption amount of the absorber was measured was subjected to centrifugal dehydration of 167 G with a centrifuge. Accordingly, the article for evaluation after centrifugation was obtained. A water retention amount (unit: g) of the absorber is a difference in a mass of the article for evaluation after centrifugation and a mass of the article for evaluation before the test, and is calculated by the following formula.

$$\text{Water retention amount of absorber} = \text{mass (g) of article for evaluation after centrifugation} - \text{mass (g) of article for evaluation before test}$$

(Evaluation of excess water content)

An excess water content is calculated by the following formula. As an excess water content becomes smaller, excess water after liquid absorption is more reduced. The results are shown in Table 1.

$$\text{Excess water content } (\%) = \{(\text{water absorption amount of absorber} - \text{water retention amount of absorber})/\text{water absorption amount of absorber}\} \times 100$$

[Table 1]

| | Performances of water-absorbent resin particle | | | | Performances of absorber | | |
|---|---|---|---|---|---|---|---|
| | Water retention amount [g/g] | Water absorption amount under load [ml/g] | Water absorption rate [sec.] | Degree of gel association [times] | Water absorption amount of absorber [g] | Water retention amount of absorber [g] | Excess water content [%] |
| Example 1 | 50 | 9 | 36 | 47 | 981 | 484 | 51 |
| Example 2 | 40 | 24 | 48 | 100 or more | 855 | 386 | 55 |
| Example 3 | 51 | 17 | 38 | 100 or more | 889 | 497 | 44 |
| Comparative Example 1 | 35 | 20 | 37 | 3 | 892 | 327 | 63 |
| Comparative Example 2 | 42 | 18 | 37 | 6 | 925 | 369 | 60 |
| Comparative Example 3 | 28 | 26 | 56 | 2 | 706 | 283 | 60 |
| Comparative Example 4 | 34 | 28 | 45 | 9 | 848 | 341 | 60 |

Based on Table 1, it was confirmed that in a case where a degree of gel association is high, an absorber in which excess water was reduced after liquid absorption could be obtained.

**Reference Signs List**

10          absorber,

10a, 65    water-absorbent resin particle,

10b         fiber layer,

20a, 20b   core wrap,

30          liquid permeable sheet,

40          liquid impermeable sheet,

61          burette unit,

| 61a | burette, |
|-----|----------|
| 61b | rubber stopper, |
| 61c | cock, |
| 61d | air introduction tube, |
| 61e | cock, |
| 62 | conduit, |
| 63 | measurement table, |
| 64 | measurement unit, |
| 64a | cylinder, |
| 64b | nylon mesh, |
| 64c | weight, |
| 70 | swollen gel, |
| 71 | graduated cylinder, |
| 72 | tubular body, |
| 73 | clamp, |
| 74 | stand, |
| 75 | saucer, |
| 76 | balance, |
| 100 | absorbent article, |
| 200 | stirring blade, |
| 200a | shaft, |
| 200b | flat plate portion, |
| Y | measurement device, |
| S | slit. |

**Claims**

1. Water-absorbent resin particles having a degree of gel association of 15 or more, the degree of gel association being measured by the following procedures (1) to (4),

    (1) 1.0 g of water-absorbent resin particles is disposed in a plastic graduated cylinder, which has a capacity of 100 ml and a mass of 35 g and includes a cylindrical part having an inner diameter of 27 mm and an outer diameter of 31 mm and a pedestal part, and then 50 ml of pure water is injected at a flow rate of 10 ml/sec to form a swollen gel,

(2) an acrylic resin tubular body, which has a circular opening with an inner diameter of 4.5 cm and is fixed in a state where both end portions are open in a vertical direction, is prepared, where a flat portion having a square outer circumference and a height of 1.0 cm is provided at an upper end portion, and the opening is located at a center of the flat portion, and ten minutes after completion of injection of the pure water, the cylindrical part of the graduated cylinder is inserted into the tubular body so that an opening part of the graduated cylinder faces vertically downward, and the pedestal part of the graduated cylinder is supported by the flat portion of the tubular body,

(3) the graduated cylinder is moved vertically upward by 10 cm $\pm$ 1 cm in a state where the tubular body is fixed, and thereafter, the graduated cylinder is freely dropped to cause the pedestal part of the graduated cylinder to collide with the flat portion of the tubular body, and

(4) the operation of (3) is performed at intervals of 10 seconds, and the number of times of causing the graduated cylinder to collide with the tubular body until 1.0 g or more of the swollen gel is dropped is measured as the degree of gel association.

2. The water-absorbent resin particles according to claim 1, wherein a water retention amount for physiological saline is 47 to 60 g/g.

3. The water-absorbent resin particles according to claim 1 or 2, wherein a water absorption rate for physiological saline is less than 70 seconds.

4. The water-absorbent resin particles according to any one of claims 1 to 3, the water-absorbent resin particles comprising a structural unit derived from at least one selected from the group consisting of (meth)acrylic acid and a salt thereof.

5. An absorber comprising the water-absorbent resin particles according to any one of claims 1 to 4.

6. An absorbent article comprising the absorber according to claim 5.

7. The absorbent article according to claim 6, wherein the absorbent article is a diaper.

Fig.1

*Fig.2*

## *Fig.3*

(A)

(B)

# Fig.4

*Fig.5*

## INTERNATIONAL SEARCH REPORT

| | International application No. |
|---|---|
| | PCT/JP2020/009505 |

**A. CLASSIFICATION OF SUBJECT MATTER**

Int.Cl. C08F8/00(2006.01)i, C08F20/06(2006.01)i, A61F13/49(2006.01)i,
A61F13/53(2006.01)i, B01J20/26(2006.01)i
FI: C08F20/06, A61F13/53300, B01J20/26D, C08F8/00, A61F13/49

According to International Patent Classification (IPC) or to both national classification and IPC

**B. FIELDS SEARCHED**

Minimum documentation searched (classification system followed by classification symbols)

Int.Cl. C08F8/00, C08F20/06, A61F13/49, A61F13/53, B01J20/26

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2005-334616 A (SUMITOMO SEIKA CHEMICALS CO., LTD.) 08.12.2005 (2005-12-08), claims, paragraphs [0001], [0022], [0030], [0054], [0060], production examples 2, 3 | 1-7 |
| X | WO 2005/063825 A1 (SUMITOMO SEIKA CHEMICALS CO., LTD.) 14.07.2005 (2005-07-14), claims, paragraphs [0001], [0023], [0032], examples 1, 3, 6 | 1-7 |
| X | WO 2012/053121 A1 (SUMITOMO SEIKA CHEMICALS CO., LTD.) 26.04.2012 (2012-04-26), claims, paragraphs [0028], [0046], [0102], example 6 | 1-7 |
| A | WO 2014/038324 A1 (SUMITOMO SEIKA CHEMICALS CO., LTD.) 13.03.2014 (2014-03-13), claims, example 5 | 1-7 |
| A | WO 2012/144564 A1 (SUMITOMO SEIKA CHEMICALS CO., LTD.) 26.10.2012 (2012-10-26), claims, examples 3, 6, 7 | 1-7 |

☒ Further documents are listed in the continuation of Box C.     ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15.05.2020 | 26.05.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2020/009505 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2004/110328 A1 (SUMITOMO SEIKA CHEMICALS CO., LTD.) 23.12.2004 (2004-12-23), claims, examples 3, 5, 10 | 1-7 |

Form PCT/ISA/210 (second sheet) (January 2015)

**INTERNATIONAL SEARCH REPORT**
Information on patent family members

International application No.

PCT/JP2020/009505

```
JP 2005-334616 A   08.12.2005    (Family: none)

WO 2005/063825 A1  14.07.2005    US 2007/0179261 A1
                                 claims, paragraphs [0001],
                                 [0040], [0050], examples 1, 3, 6
                                 CN 1898270 A
                                 EP 1714985 A1
                                 KR 10-2007-0003831 A

WO 2012/053121 A1  26.04.2012    US 2013/0260151 A1
                                 claims, paragraphs [0046],
                                 [0077], [0167], example 6
                                 CN 103154043 A
                                 EP 2631251 A1
                                 KR 10-2013-0140723 A

WO 2014/038324 A1  13.03.2014    US 2015/0216740 A1
                                 claims, example 5
                                 CN 104507565 A
                                 EP 2893974 A1
                                 KR 10-2015-0054796 A

WO 2012/144564 A1  26.10.2012    US 2014/0031203 A1
                                 claims, examples 3, 6, 7
                                 CN 103502287 A
                                 EP 2700663 A1

WO 2004/110328 A1  23.12.2004    US 2007/0093766 A1
                                 claims, examples 3, 5, 10
                                 CN 1805723 A
                                 EP 1637105 A1
```

Form PCT/ISA/210 (patent family annex) (January 2015)

**EP 3 936 529 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H6345819 A
- JP H9510889 W